Europäisches Patentamt

(19) European Patent Office

Office européen des brevets

(11) Publication number: **0 028 815**
**B1**

(12) **EUROPEAN PATENT SPECIFICATION**

(45) Date of publication of patent specification: **18.06.86**

(21) Application number: **80106852.9**

(22) Date of filing: **06.11.80**

(51) Int. Cl.⁴: **A 61 K 39/39**, A 61 K 37/02

(54) **The use of ricin and a pharmaceutical composition containing the same for increasing the immune response.**

(30) Priority: **09.11.79 JP 144327/79**

(43) Date of publication of application:
**20.05.81 Bulletin 81/20**

(45) Publication of the grant of the patent:
**18.06.86 Bulletin 86/25**

(84) Designated Contracting States:
**BE CH DE GB LI NL SE**

(56) References cited:

PATENTS ABSTRACTS OF JAPAN, vol. 2, no.
85, July 12, 1978, C-Section, page 1255 C 78
EXPERIENTIA, vol. 27, March 15, 1971,
"Adjuvant activity of ricin as measured by
Jern's plaque technique", pages 323-324
Nature, vol. 277, pages 292-293
Burger's Medicinal Chemistry, 4th Ed., Part II,
pages 694-697

(73) Proprietor: **Eisai Co., Ltd.**
**6-10, Koishikawa 4-chome Bunkyo-ku**
**Tokyo 112 (JP)**

(72) Inventor: **Shionoya, Hiroshi**
**861-33, Kamiarai**
**Tokorozawa-shi Saitama Pref. (JP)**
Inventor: **Arai, Haruyoshi**
**24-4, Aza-Todomegi, Oh-aza-Hashizume**
**Inuyama-shi Aichi Pref. (JP)**
Inventor: **Koyanagi, Nozomu**
**3-14-7, Kitano**
**Niiza-shi Saitama Pref. (JP)**
Inventor: **Takeuchi, Hitoshi**
**3-2-5, Nakamura**
**Nerima-ku Tokyo (JP)**

(74) Representative: **Hansen, Bernd, Dr.rer.nat. et al**
**Hoffmann, Eitle & Partner Patentanwälte**
**Arabellastrasse 4**
**D-8000 München 81 (DE)**

Courier Press, Leamington Spa, England.

**Description**

The invention concerns the use of ricin and a pharmaceutical composition containing the same for increasing the immune response.

Ricin used in this invention is a lectin isolated from the seeds of castor beans which is a plant of the Euphorbiaceae; it is a type of glycoprotein having a molecular weight of about 65,000.

Immune protection in vertebrates against infections is known to be provided by two basic immune response systems. One is the cellular immune response system effective mainly against infections by fungi, viruses, intracellular parasitic bacteria, etc., and the other is the humoral immune response system effective for defence mainly against infections by viruses and extracellular parasitic bacteria.

A substance, which when injected into a living organism together with an antigen, increases the aforesaid immune responses to the antigen is known as an immuno-adjuvant [Davis, B. D. et al., Microbiology, Harper & Row Publishers, page 458, (1967)]. Heretofore, the Freund's complete adjuvant has been used mainly as the immuno-adjuvant. Since, however, Freund's complete adjuvant is composed of killed Mycobacteria, a surface-active agent and a mineral oil, a complex operation is required for formulating it into a dosage form by mixing it with an antigen in the form of an aqueous solution, a suspension, etc., and moreover, strong necrosis or swelling occurs at the injected site. Accordingly, it is not possible to use Freund's complete adjuvant in humans.

JP—A—53 44 613 describes a tumor vaccine useful as remedy for cancer. This tumor vaccine may for instance consist of a ricin/tumor-cell complex. This complex is prepared by adding ricin to suspended tumor cells according to a well-defined procedure.

The publication in Experimentia, Vol. 27, pages 323—324, relates to "Adjuvant Activity of Ricin as measured by Jerne's Plaque Technique". This reference describes the use of ricin as immunological adjuvant together with a red blood cell suspension. This document does not describe an administration of the immunological adjuvant together with a red blood cell suspension for a prophylactic or curative effect. The sole purpose of the adjuvant ricin is to increase the output of antibody.

It is an object of this invention to use an immunopotentiator having the ability to increase both cellular and humoral immune responses and to provide a pharmaceutical composition therefor.

Accordingly, this invention discloses the use of ricin for the manufacture of a medicament for increasing the immune response to intracellular or extracellular parasitic bacteria in humans or animals.

Further, the invention provides a pharmaceutical composition suitable for parenteral administration comprising ricin, an antigen and a pharmaceutically acceptable diluent.

Ricin used as the active ingredient of the immunopotentiator of this invention is an immuno-adjuvant having the ability to potentiate both the aforesaid cellular and humoral immune responses. It is characterized by having a strong adjuvant activity in amounts which do not cause necrosis, swelling, etc. at the site of injection. The ricin in accordance with this invention also has the advantage that since it can be used in the form of an aqueous solution, it can be easily formulated into a dosage form by mixing with an antigen.

As to the acute toxicity of ricin used in this invention, experiments of the present inventors showed that $LD_{50}$ of ricin is 12 µg/kg (mouse, i.p.).

The minimum dosage of ricin which shows an immunopotentiating activity is about 0.2 ng per human or animal irrespective of body weight. The maximum dosage is generally about 1 to about 1.5 µg/kg. These dosages are much lower than the lethal dose mentioned above.

Ricin in accordance with this invention may be administered together with various antigens, and the preferred route of administration is subcutaneous, intramuscular or intraperitoneal. Furthermore, ricin can be administered separately from an antigen, and in this case, intravenous and intrapleural administrations are possible in addition to the aforesaid administration routes. The administrations of ricin are preferably twice a week to once in two weeks.

For administration, in accordance with this invention the ricin may be formulated into any desired injectable form by known formulating methods. For example, it may be in the form of an injecting ampoule containing 0.1 to 1 ml of a physiological saline or a neutral to weakly acidic buffer containing ricin in a concentration of about 100 µg to 1 mg/ml, or an ricin-containing lyophilized ampoule.

The immunopotentiating activity of ricin is illustrated specifically by the following Examples.

Example 1
Immunopotentiative effect of ricin on production of humoral antibody in mice

The effect of ricin on the production of a humoral antibody in mice was examined using bovine serum albumin (BSA) as an antigen.

A solution (0.2 ml) obtained by mixing 0.1 ml of a physiological saline containing 1 µg of BSA antigen with 0.1 ml of a physiological saline containing ricin in various amounts from 0.1 ng to 30 ng was subcutaneously injected into the back of mice (female CDF 1) having a body weight of 20 to 23 g (primary immunization). Two weeks later, the same solution was injected into the mice in the same manner (secondary immunization). Twelve days after the secondary immunization, blood was taken from each of the mice, and the plasma was separated.

The antibody titer of the plasma was measured in accordance with a method of hemagglutination of tanned sheep red blood cells passively sensitized with BSA [Proteins, Nucleic Acids, Enzymes; separate print "Immunobiochemistry", Vol. 11, No. 15, page 1506, (1966)]. The results are shown in Table 1.

TABLE 1

Activity of ricin to increase production of an anti-BSA antibody

| Amount of ricin (ng/mouse) | Number of animals | Number of mice having plasma HA titer estimated by BSA-sensitized sheep red blood cell hemagglutination reaction HA titer * | | | | | |
|---|---|---|---|---|---|---|---|
| | | 0 | 20 | 40 | 80 | 160 | 320 |
| 0 | 10 | 10 | | | | | |
| 0.1 | 4 | 3 | | | | | 1 |
| 0.3 | 5 | 3 | | 2 | | | |
| 1 | 4 | 1 | 1 | 1 | 1 | | |
| 3 | 5 | | | 2 | 1 | 2 | |
| 10 | 5 | | | | | 2 | 3 |
| 30 | 5 | 2 | 1 | 1 | 1 | | |

* HA titer=hemagglutination titer.

As is apparent from Table 1, immunopotentiative activity of ricin on humoral antibody product was recognized in the range of 1 ng—30 ng per mouse and was in the maximum at 10 ng.

Example 2

Immunopotentiative effect of ricin on cellular immune response in mice

A physiological saline (0.2 ml) containing 1 μg of BSA and various amounts of ricin was injected subcutaneously into the back of mice (female CDF 1) having a body weight of 20 to 23 g and being 8—10 weeks old, and two weeks later, the mice were treated with injection in the same way as above (secondary immunization). Twenty-seven days after the secondary immunization, 10 μl of a physiological saline containing 10 μg of BSA was injected subcutaneously into the auricle of each mouse (challenge), in accordance with the method of J. H. Robinsons et al. [Scand. J. Immunol., 5, 299 (1976)]. Twenty-four hours later, an ear swelling was measured. The results are shown in Table 2.

TABLE 2
Cellular immuno-potentiative activity of ricin

| Groups | Number of animals | Immunization | | Challenge BSA (μg/mouse) | Ear swelling ($10^{-3}$ cm±S.E.*) | Significance against control (p) |
| --- | --- | --- | --- | --- | --- | --- |
| | | Ricin (ng/mouse) | BSA (μg/mouse) | | | |
| Control | 6 | 0 | 1 | 10 | 5.0±1.2 | — |
| 1 | 4 | 0.3 | 1 | 10 | 8.8±1.9 | <0.05 |
| 2 | 4 | 1 | 1 | 10 | 8.3±1.3 | <0.05 |
| 3 | 5 | 3 | 1 | 10 | 11.0±1.5 | <0.01 |
| 4 | 5 | 10 | 1 | 10 | 16.8±1.6 | <0.01 |
| 5 | 5 | 30 | 1 | 10 | 10.2±1.9 | <0.01 |

* S.E.=standard error.

The results of Table 2 reveals that ricin used in an amount ranging from 3 ng to 30 ng enhances the cellular immune response against BSA.

Example 3
Preparation of an ricin-containing ampoule

Glacial acetic acid (0.05 ml) was added on ice water bath to 5 ml of an aqueous suspension containing ricin crystals in a concentration of 10 mg/ml to dissolve the ricin crystals. To the resulting solution was added 10 ml of a 0.01M phosphate buffer (pH 6.0) containing 0.15M of sodium chloride [to be abbreviated as PBS (pH 6.0)]. Then, 0.4 ml of a 1M $Na_2HPO_4$ solution was added to adjust the pH of the solution to about 5. The solution was centrifuged at 15000 rpm for 10 minutes. The supernatant solution was filtered by a 0.45 µm filter (Millipore® Filter, type HA) to remove microbes. The filtrate was put into a cellophane tube, and dialyzed overnight against 5 liter of PBS (pH 6.0).

The dialyzate was diluted with PBS (pH 6.0) to form a solution containing ricin in a concentration of 500 µg/ml). The concentration of ricin was adjusted according to its $E_{280}^{1\%}$ nm value of 11.8 reported by Olsnes et al. [J. Biol. Chem., *249*, 803—810 (1974)]. The ricin solution was put separately in an amount of 0.1 ml into each of 1 ml glass ampoules to prepare injecting ampoules each containing 50 µg of ricin.

## Claims

1. The use of ricin for the manufacture of a medicament for increasing the immune response to intracellular or extracellular parasitic bacteria in humans or animals.

2. The use of ricin for the manufacture of a medicament in a parenterally administrable form for increasing the immune response to intracellular or extracellular parasitic bacteria in humans or animals.

3. The use of ricin for the manufacture of a medicament in a parenterally administrable form containing at least 0,2 ng of ricin for increasing the immune response to intracellular or extracellular parasitic bacteria in humans or animals.

4. A pharmaceutical composition suitable for parenteral administration comprising ricin, an antigen and a pharmaceutically acceptable diluent.

5. A pharmaceutical composition according to claim 4 wherein the diluent is water.

## Patentansprüche

1. Verwendung von Ricin zur Herstellung eines Arzneimittels zur Erhöhung der Immunantwort auf intrazelluläre oder extrazelluläre parasitische Bakterien bei Menschen und Tieren.

2. Verwendung von Ricin zur Herstellung eines Arzneimittels in parenteral verabreichbarer Form zur Erhöhung der Immunantwort auf intrazelluläre oder extrazelluläre parasitische Bakterien bei Menschen oder Tieren.

3. Verwendung von Ricin zur Herstellung eines Arzneimittels in parenteral verabreihbarer Form, welches mindestens 0,2 ng Ricin enthält, zur Erhöhung der Immunantwort auf intrazelluläre oder extrazelluläre parasitische Bakterien bei Menschen oder Tieren.

4. Pharmazeutische Zubereitung zur parenteralen Verabreichung, welches Ricin, ein Antigen und ein pharmazeutisch annehmbares Verdünnungsmittel enthält.

5. Pharmazeutische Zubereitung nach Anspruch 4, wobei das Verdünnungsmittel Wasser darstellt.

## Revendications

1. Utilisation de la ricine pour la fabrication d'un médicament pour augmenter la réponse immune vis-à-vis des bactéries parasites intracellulaires ou extracellulaires chez l'homme ou les animaux.

2. Utilisation de la ricine pour la fabrication d'un médicament administrable sous forme parentérale pour augmenter la réponse immune vis-à-vis des bactéries parasites intracellulaires ou extracellulaires chez l'homme ou les animaux.

3. Utilization de la ricine pour la fabrication d'un médicament administrable sous forme parentérale contenant au moins 0,2 ng de ricine pour augmenter la réponse immune vis-à-vis des bactéries parasites intracellulaires ou extracellulaires chez l'homme ou les animaux.

4. Composition pharmaceutiques convenant à l'administration parentérale comprenant de la ricine, un antigène et un diluant pharmaceutiquement acceptable.

5. Composition pharmaceutique selon la revendication 4, caractérisée en ce que le diluant est l'eau.